(19) Europäisches Patentamt
European Patent Office
Office européen des brevets

(11) Veröffentlichungsnummer: **0 179 378**
**B1**

(12) # EUROPÄISCHE PATENTSCHRIFT

(45) Veröffentlichungstag der Patentschrift: **06.12.89**

(21) Anmeldenummer: **85112987.4**

(22) Anmeldetag: **14.10.85**

(51) Int. Cl.⁴: **C 07 D 265/18**, C 07 D 413/04, B 41 M 5/12, C 09 B 11/26, C 09 B 11/24, C 09 B 11/06, C 09 B 11/04, C 07 C 127/22, C 07 C 125/065 // (C07D413/04, 265:00, 231:00)

(54) **Chromogene 4,4-Diaryl-2-oxo-benzo-3,1-oxazine, ihre Herstellung und Verwendung.**

(30) Priorität: **26.10.84 DE 3439281**

(43) Veröffentlichungstag der Anmeldung: **30.04.86 Patentblatt 86/18**

(45) Bekanntmachung des Hinweises auf die Patenterteilung: **06.12.89 Patentblatt 89/49**

(84) Benannte Vertragsstaaten: **CH DE FR GB LI**

(56) Entgegenhaltungen:
**US-A-4 074 050**

**CHEMICAL ABSTRACTS, Band 101, Nr. 7, 13. August 1984, Seite 616, Nr. 55014s, Columbus, Ohio, US; B.K. MISRA et al.: "Friedel-crafts acylation of some aromatic compounds with 2-isocyanatobenzoyl chloride"**

**Die Akte enthält technische Angaben, die nach dem Eingang der Anmeldung eingereicht wurden und die nicht in dieser Patentschrift enthalten sind.**

(73) Patentinhaber: **BAYER AG, D-5090 Leverkusen 1 Bayerwerk (DE)**

(72) Erfinder: **Meisel, Karlheinrich, Dr., Carl- von-Ossietzky- Strasse 24, D-5090 Leverkusen (DE)**
Erfinder: **Berneth, Horst, Dr., Erfurter Strasse 1, D-5090 Leverkusen (DE)**

**EP 0 179 378 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

**Beschreibung**

Gegenstand der Erfindung sind chromogene 4,4-Diaryl-2-oxo-benzo-3,1-oxazine der allgemeinen Formel

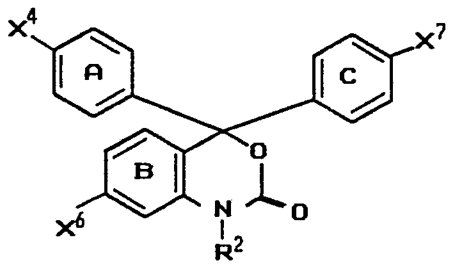

(I)

worin

$X^4$, $X^6$ und $X^7$ unabhängig voneinander Wasserstoff, Halogen, $C_1$ - bis $C_{18}$-Alkyl, gegebenenfalls durch Chlor und/oder $C_1$- bis $C_{12}$-Alkyl substituiertes Phenyl, $C_1$- bis $C_{12}$-Alkylcarbonylamino, gegebenenfalls durch Chlor und/oder $C_1$- bis $C_{12}$-Alkyl substituiertes Benzoylamino, $NY^4Y^5$, $OY^6$ oder $SY^6$, 2- oder 4-Pyridyl, 2-, 4- oder 5-Pyrimidyl, Pyrazolinyl, 3-Indolyl oder 4-Piperidyl, die durch $C_1$- bis $C_4$-Alkyl oder Phenyl substituiert sein können, wobei Phenyl seinerseits durch Amino, $C_1$- bis $C_{12}$-Mono- oder -Dialkylamino, Halogen, $C_1$- bis $C_{12}$-Alkoxy oder $C_1$-$C_{12}$-Alkyl substituiert sein kann,

wobei wenigstens einer der Reste

$X^4$, $X^6$ oder $X^7$ für $NY^4Y^5$ steht,
$R^2$ Wasserstoff, $C_1$- bis $C_4$-Alkyl, Cyclohexyl oder gegebenenfalls durch Chlor und/oder $C_1$- bis $C_4$-Alkyl substituierte Benzyl - oder Phenylreste,
$Y^4$, $Y^5$ und $Y^6$ unabhängig voneinander Wasserstoff, $C_1$-$C_{18}$-Alkyl, das durch Chlor, Cyano, $C_1$- bis $C_{12}$-Alkoxycarbonyl oder $C_1$- bis $C_{12}$-Alkoxy substituiert sein kann, Cyclohexyl, Phenyl oder Benzyl die durch Chlor, Hydroxy, $C_1$- bis $C_{12}$-Alkyl oder $C_1$- bis $C_{12}$-Alkoxy substituiert sein können, oder Glieder die zusammen mit N oder O, an das sie gebunden sind, und mit einem der Ringe A, B oder C zur Vervollständigung eines Ringsystems der folgenden Formeln

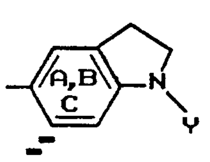 , 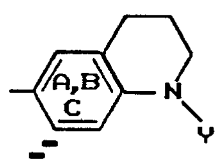 , 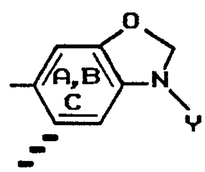 , 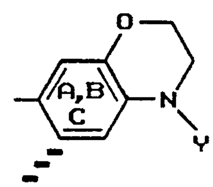

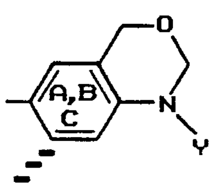 , 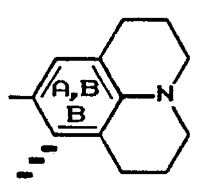 , 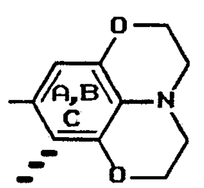 , 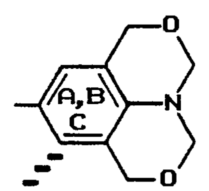 ,

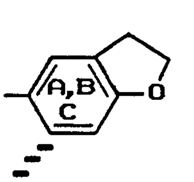 , 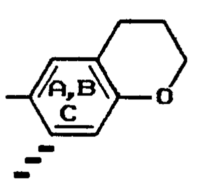 , 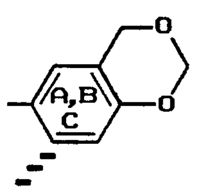 , 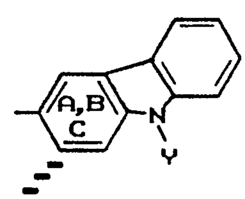

erforderlich sind, bedeuten, worin
die gestrichelte Linie die weitere Anellierung im Fall von Ring B bedeutet,

Y für Wasserstoff, gegebenenfalls durch Chlor, Cyano, $C_1$- bis $C_4$-Alkoxycarbonyl oder $C_1$- bis $C_4$-Alkoxy substituiertes $C_1$- bis $C_8$-Alkyl, Cyclohexyl oder Phenyl oder Benzyl, die durch Chlor, $C_1$- bis $C_4$-Alkyl oder $C_1$- bis $C_4$-Alkoxy substituiert sein können, steht,

der gesättigte Ringteil bis zu 4 Reste aus der Gruppe Chlor, $C_1$- bis $C_4$-Alkyl, $C_1$- bis $C_4$-Alkoxy und Phenyl tragen kann,

die Ringe A, B, C durch Chlor, $C_1$- bis $C_4$-Alkyl, $C_1$- bis $C_4$-Alkoxy und/oder $C_1$- bis $C_{12}$-Alkanoylamino substituiert sein können, oder

$NY^4Y^5$ einen gegebenenfalls durch Chlor, $C_1$ - bis $C_{18}$-Alkyl, Amino-$C_1$-$C_4$-Alkyl oder Phenyl substituierten Pyrrolo-, Pyrrolidino-, Piperidino-, Pipecolino-, Morpholino-, Pyrazolo- oder Pyrazolinorest bedeuten, ihre Herstellung und ihre Verwendung in druckkopierfähigen und thermoreaktiven Aufzeichnungsmaterialien.

Unter Halogen ist im besonderen Chlor und Brom zu verstehen.

Bevorzugt sind Verbindungen der Formel

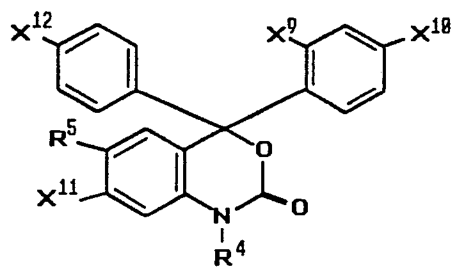

(II)

worin wenigstens einer der Reste

$X^{10}$, $X^{11}$ oder $X^{12}$ $NY^7Y^{7'}$ und die übrigen unabhängig voneinander $NY^7Y^{7'}$, Wasserstoff, Halogen, $C_1$- bis $C_{18}$-Alkyl, $C_1$- bis $C_{12}$-Alkoxy, Benzyloxy, Methyl- oder Ethylmercapto, Phenyl oder Pyrazolino, das durch $C_1$- bis $C_4$-Alkyl oder Phenyl substituiert sein kann,
$X^9$ Wasserstoff, $C_1$- bis $C_4$-Alkyl, $C_1$- bis $C_4$-Alkoxy, Benzyloxy, Methyl- oder Ethylmercapto,
$Y^7$ und $Y^{7'}$ unabhängig voneinander Wasserstoff, $C_1$- bis $C_{18}$-Alkyl, das durch Cyan, Chlor, $C_1$- bis $C_3$-Alkoxy oder $C_1$- bis $C_3$-Alkoxycarbonyl substituiert sein kann oder dessen Substituenten zu einem Piperidin-, Morpholin-, Piperazinring geschlossen sein können, Benzyl oder Phenyl, die durch Chlor, Hydroxy, $C_1$- bis $C_4$-Alkyl , $C_1$- bis $C_4$-Alkoxy, Cyan oder $C_1$- bis $C_4$-Alkoxycarbonyl substituiert sein können, bedeuten, und
$R^4$ und $R^5$ unabhängig voneinander für Wasserstoff, $C_1$- bis $C_4$-Alkyl Cyclohexyl, Benzyl oder Phenyl stehen.

Ebenfalls bevorzugt sind Verbindungen der allgemeinen Formel

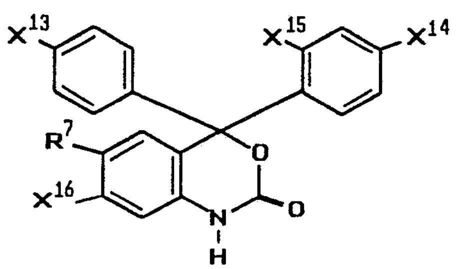

(III)

worin

$R^7$ Wasserstoff, $C_1$- bis $C_4$-Alkyl oder Benzyl bedeutet,

wenigstens einer der Reste
$X^{13}$, $X^{14}$ oder $X^{16}$ für $NY^9Y^{9'}$ steht und die übrigen unabhängig voneinander Wasserstoff, $NY^9Y^{9'}$, Halogen, $C_1$-$C_{18}$-Alkyl, $C_1$-$C_{12}$-Alkoxy, Benzyloxy, Methylmercapto oder Ethylmercapto, Phenyl oder Pyrazolino, das durch $C_1$- bis $C_4$-Alkylgruppen oder Phenylgruppen weiter substituiert sein kann,
$X^{15}$ Wasserstoff, $C_1$- bis $C_3$-Alkoxy, Methylmercapto oder Ethylmercapto,
$Y^9$ und $Y^{9'}$ unabhängig voneinander Wasserstoff, $C_1$- bis $C_{18}$-Alkyl, das durch Cyano, Chlor, $C_1$- bis $C_3$-Alkoxy oder $C_1$- bis $C_3$-Alkoxycarbonyl substituiert sein kann, oder Benzyl, das durch Chlor, $C_1$- bis $C_4$-Alkyl oder $C_1$- bis $C_4$-Alkoxy, substituiert sein kann, oder

$Y^{9'}$ einen Rest der Formel 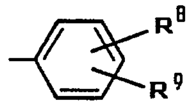 bedeuten,

worin

$R^8$ und $R^9$ unabhängig voneinander Chlor, $C_1$- bis $C_4$-Alkyl, $C_1$- bis $C_4$-Alkoxy, Phenyloxy, Benzyloxy, Cyano, Pyrrolidino, Piperidino, Morpholino, Piperazino, Pyrazolino oder Imidazolino bedeuten.

Ein weiterer Gegenstand der Erfindung sind Verbindungen der allgemeinen Formel

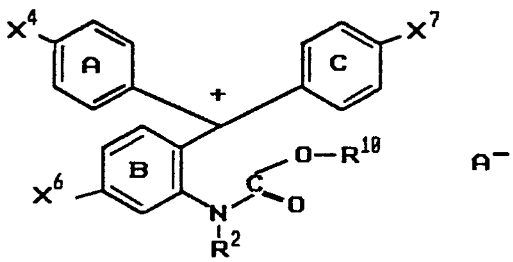

(IV)

worin

$X^4$, $X^6$, $X^7$, $R^2$, A, B und C die oben angegebene Bedeutung haben,
$A^-$ ein Anion bedeutet und
$R^{10}$ für $C_1$-$C_4$-Alkyl oder Phenyl steht.

Die Herstellung der oben aufgeführten Farbbildner erfolgt dadurch, daß man Farbstoffe der Formel IV auf übliche Weise in wäßrigem und/oder nicht-wäßrigem alkalischem Medium gegebenenfalls unter Zusatz eines Phasentransferkatalysators als Lösungsvermittler bei Temperaturen zwischen 0°C und dem Siedepunkt des betreffenden Reaktionsmediums umsetzt.

Anschließend wird evtl. nach Entfernen des nichtwäßrigen Lösungsmittels, auf beispielsweise Wasser oder einen Alkohol ausgetragen. Das abgeschiedene Produkt wird abgesaugt und mit Wasser einige Stunden nachgerührt.

Die Farbstoffe der Formel (IV) erhält man vorzugsweise durch Kondensation eines entsprechend substituierten Phenylcarbaminsäureesters der Formel

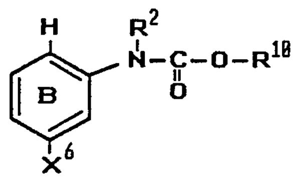

(V)

mit einem Keton der Formel

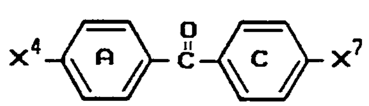

(VI)

worin

$X^4$, $X^6$, $X^7$, $R^2$, $R^{10}$, A, B und C die oben angegebene Bedeutung besitzen.

Insbesondere handelt es sich bei den Verbindungen (V) um solche, bei denen

$X^6$ ein Elektronendonorsubstituent ist, wie $NY^4Y^5$, $OY^6$ oder $SY^6$, worin

$Y^4$ bis $Y^5$ die oben angegebene Bedeutung haben und der Ring B nicht durch starke Elektronenakzeptorgruppen wie Nitro, Cyano oder Alkoxycarbonyl desaktiviert ist.

Die Umsetzung erfolgt üblicherweise mit wasserabspaltenden Reagenzien ohne oder auch mit unter diesen Bedingungen inerten Lösungsmitteln bei Temperaturen zwischen 0°C und dem Siedepunkt des jeweiligen Mediums. Anschließend wird evtl. nach Entfernen der inerten Lösungsmittel auf beispielsweise Wasser oder einen Alkohol ausgetragen.

Wasserabspaltende Reagenzien sind beispielsweise Phosphoroxychlorid, Phosphorpentachlorid Diphosphorpentoxid Phosgen, Phosphortrichlorid, Phosphortribromid, Sulfurylchlorid, Thionylchlorid, Oxalylchlorid oder Mischungen hiervon. Vorzugsweise werden Phosphoroxychlorid und Phosphoroxychlorid/Diphosphorpentoxid eingesetzt.

Geeignete inerte Lösungsmittel sind beispielsweise Toluol, Chlorbenzol, Dichlorbenzol, Nitrobenzol, chlorierte aliphatische Kohlenwasserstoffe, wie 1,2-Dichlor-ethan.

Die Herstellung der Farbstoffe der Formel (IV) kann auch durch Oxidation von Leukoverbindungen der Formel

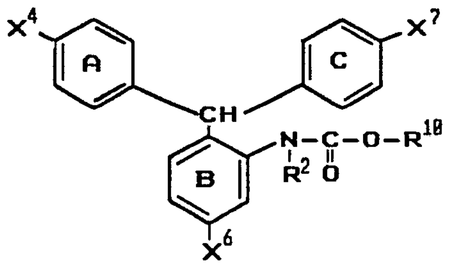

(VII)

worin

$X^4$, $X^6$, $X^7$, $X^2$, $R^{10}$, A, B und C die oben angegebene Bedeutung haben, erfolgen.

Diese Oxidation kann auf bekannte Weise mit höherwertigen Metallverbindungen, wie $PbO_2$, $MnO_2$, Permanganaten $CrO_3$, Chromaten, Dichromaten, $NiO_2$, $K_3$ $[Fe(CN)_6]$, mit Chinonen, wie Chloranil, Tetrachlor-o-chinon, Dichlordicyanochinon, oder auf eine andere literaturbekannte Weise, wie z. B. mit Sauerstoff, Luft, Perboraten oder Wasserstoffperoxid, erfolgen.

Die Aufarbeitung, Isolierung und evtl. Nachbehandlung erfolgt auf analoge Weise wie oben beschrieben.

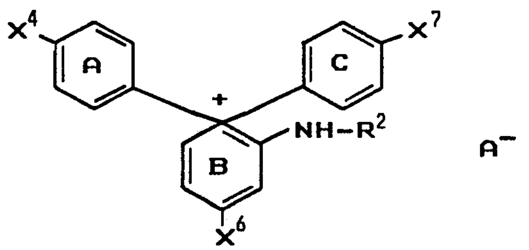

(VIII)

worin

$X^4$, $X^6$, $X^7$, $R^2$, A, B und C die oben aufgeführte Bedeutung haben und
$A^-$ ein Anion bedeutet,

mit Phosgen oder seinen Derivaten umsetzt.

Als Derivate des Phosgens seien besonders genannt: Chlorameisensäureethylester Chlorameisensäurephenylester, Dimethylcarbonat, Diphenylcarbonat.

Die Umsetzung erfolgt zweckmäßigerweise in wäßrigem und/oder nichtwäßrigem alkalischem Medium bei Temperaturen zwischen -10°C und +40°C. Unter Umständen ist der Zusatz einer Base, z. B. Natriumcarbonat oder Natriumhydroxid und gegebenenfalls der Zusatz eines Phasentransferkatalysators als Lösungsvermittler sinnvoll.

Als nichtwäßrige Lösungsmittel seien besonders genannt: Toluol, Xylol, Chlorbenzol, Dichlorbenzol, Pyridin Ethylenglykoldimethylether, Chloroform, Tetrachlorkohlenstoff, Dichlorethan, Tetrachlorethan oder Alkohole, z. B. Methanol, Ethanol, i-Propanol.

Die Oxidation mit höherwertigen Metallverbindungen erfolgt üblicherweise in saurem Medium oder in organischen Lösungsmitteln, wie Alkoholen- z. B. Ethanol, Isopropanol, Ethylenglykolmonomethylether; Ketonen - z. B. Aceton, Butanon oder Methylisopropylketon oder polaren aprotischen Lösungsmitteln, z. B. N-Methyl-pyrrolidon, γ-Butyrolacton, Acetonitril, Dimethylsulfoxid oder Sulfolan oder in Mischungen solcher Lösungsmittel mit Säuren bei Temperaturen zwischen 0°C und 60°C, vorzugsweise 10 - 40°C.

Geeignete Säuren sind beispielsweise Salzsäure, Schwefelsäure, Phosphorsäure, Essigsäure, Propionsäure oder Mischungen untereinander und/oder Mischungen mit Wasser. Eine bevorzugte Mischung ist Salzsäure, Essigsäure und Wasser.

Die Oxidation mit Chinonen erfolgt üblicherweise in organischen Lösungsmitteln, wie Alkoholen - z. B. Methanol, Ethanol, Isopropanol; Ketonen - z. B. Aceton, Butanon; Estern z. B. Essigsäureethyl- oder -butylester;

Carbonsäuren - z. B. Essigsäure, Propionsäure oder polaren aprotischen Lösungsmitteln, wie N-Methylpyrrolidon, Dimethylformamid, γ-Butyrolacton, Acetonitril, Sulfolan oder aber in Mischungen hiervon bei Temperaturen zwischen 0°C und dem Siedepunkt des Mediums, vorzugsweise 20 - 70°C.

Ein weiteres Verfahren zur Herstellung der erfindungsgemäßen Verbindungen besteht darin, daß man Farbstoffe der allgemeinen Formel

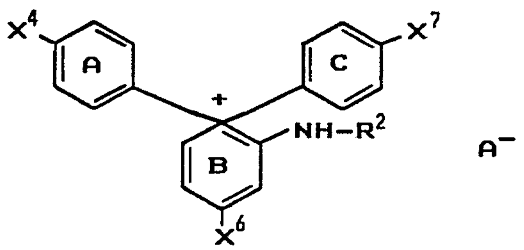

(VIII)

worin

$X^4$, $X^6$, $X^7$, $R^2$, A, B und C die oben aufgeführte Bedeutung haben und

$A^-$ ein Anion bedeutet,

mit Phosgen oder seinen Derivaten umsetzt.

Als Derivate des Phosgens seien besonders genannt:

Chlorameisensäureethylester, Chlorameisensäurephenylester, Dimethylcarbonat, Diphenylcarbonat.

Die Umsetzung erfolgt zweckmäßigerweise in wäßrigem und/oder nichtwäßrigem alkalischem Medium bei Temperaturen zwischen -10°C und +40°C. Unter Umständen ist der Zusatz einer Base, z. B. Natriumcarbonat oder Natriumhydroxid und gegebenenfalls der Zusatz eines Phasentransferkatalysators als Lösungsvermittler sinnvoll.

Als nichtwäßrige Lösungsmittel seien besonders genannt:

Toluol, Xylol, Chlorbenzol, Dichlorbenzol, Pyridin, Ethylenglykoldimethylether, Chloroform, Tetrachlorkohlenstoff, Dichlorethan, Tetrachlorethan oder Alkohole, z. B. Methanol, Ethanol, i-Propanol.

Als Basen sind bevorzugt zu nennen:

Carbonate, wie z. B. Natriumcarbonat, Kaliumcarbonat, Calciumcarbonat; Oxide wie z. B. Magnesiumoxid oder Calciumoxid; Amine, wie z. B. Triethylamin, Pyridin.

Die Aufarbeitung der Produkte erfolgt gegebenenfalls nach Entfernen des inerten Lösungsmittels wie oben beschrieben.

Die Verbindungen der Formel (I) sind normalerweise farblos oder höchstens schwach gefärbt.

Wenn diese Farbbildner mit einem vorzugsweise sauren Entwickler, d. h. einem Elektronenakzeptor, in Kontakt gebracht werden, so ergeben sich intensive rote, blaue, grüne oder schwarze Farbtöne, die ausgezeichnete Echtheiten aufweisen.

Sie sind auch wertvoll im Gemisch mit einem oder mehreren anderen bekannten Farbbildnern, z. B. 3,3-Bis-(aminophenyl)-phthaliden, 3,3-Bis-(indolyl)-phthaliden, 3-Amino-fluoranen, 2,6-Diamino-fluoranen, Leukoauraminen, Spiropyranen, Spirodipyranen, Chromenoindolen, Phenoxazinen, Phenothiazinen, Carbazolylmethanen oder weiteren Triarylmethanleukofarbstoffen.

Die Verbindungen der Formel (I) zeigen sowohl auf phenolischen Unterlagen wie auch besonders auf aktivierten Tonen eine gute Farbintensität und Lichtechtheit. Sie eignen sich vor allem als Farbbildner für die Verwendung in einem wärmeempfindlichen oder druckempfindlichen Aufzeichnungsmaterial, das sowohl Kopierals auch Registriermaterial sein kann. Ihre Entwicklungsgeschwindigkeit ist in Anhängigkeit von den Substituenten unterschiedlich. Eine geringe Entwicklungsgeschwindigkeit führt zu einer reduzierten Empfindlichkeit der Aufzeichnungsmaterialien gegenüber unbeabsichtigter vorzeitiger Entwicklung.

Insbesondere beim Thermodruck-Verfahren sind mit den erfindungsgemäßen Farbbildnern Schriftbilder extrem hoher Echtheiten und hoher Unempfindlichkeit gegen den Einfluß sowohl saurer als auch basischer Medien erhältlich.

Ein druckempfindliches Material besteht beispielsweise aus mindestens 1 Paar von Blättern, die mindestens einen Farbbildner der Formel I, gelöst oder dispergiert in einem nichtflüchtigen organischen Lösungsmittel, und einen Elektronenakzeptor als Entwickler enthalten.

Typische Beispiele für solche Entwickler sind anorganische Stoffe wie Tone, Metallsalze oder -oxide oder organische Polymerisate wie Phenolharze.

Die Entwickler können zusätzlich auch im Gemisch mit anderen an sich unreaktiven oder wenig reaktiven Pigmenten eingesetzt werden.

Der Farbbildner liefert an den Punkten, an denen er mit dem Elektronenakzeptor in Kontakt kommt, eine gefärbte Markierung. Um eine frühzeitige Aktivierung der in dem druckempfindlichen Aufzeichnungsmaterial vorhandenen Farbbildner zu verhindern, werden diese in der Regel von dem Elektronenakzeptor getrennt. Dies kann zweckmäßig erzielt werden, indem man die Farbbildner in schaum-, schwamm- oder bienenwabenartigen Strukturen einarbeitet. Vorzugsweise sind die Farbbildner in Mikrokapseln eingeschlossen, die sich in der Regel

durch Druck zerbrechen lassen. Verfahren zur Herstellung derartiger Mikrokapseln sind bekannt.

Als nichtflüchtige Lösungsmittel sind z. B. partiell hydriertes Terphenyl, alkylierte Naphthaline oder Dibutylphthalat geeignet.

Bevorzugt wird eine Anordnung, bei der der eingekapselte Farbbildner in Form einer Schicht auf der Rückseite eines Übertragungsblattes und der Elektronenakzeptor in Form einer Schicht auf der Vorderseite eines Empfangsblattes vorhanden sind.

Eine andere Anordnung der Bestandteile besteht darin, daß die den Farbbildner enthaltenden Mikrokapseln und der Entwickler in oder auf dem gleichen Blatt in Form einer oder mehrerer Einzelschichten oder in der Papierpulpe vorliegen.

Die Verbindungen der Formel I können vorzugsweise auch als Farbbildner in einem thermoreaktiven Aufzeichnungsmaterial verwendet werden. Dieses enthält in der Regel mindestens einen Schichtträger, einen Farbbildner, einen Elektronenakzeptor und gegebenenfalls auch ein Bindemittel.

Thermoreaktive Aufzeichnungssysteme umfassen z. B. wärmeempfindliche Aufzeichnungs und Kopiermaterialien und -papiere. Diese Systeme werden beispielsweise zum Aufzeichnen von Informationen, z. B. in elektronischen Rechnern, Ferndruckern, Fernschreibern oder in Aufzeichnungsgeräten und Meßinstrumenten, wie z. B. Elektrokardiographen, verwendet. Die Bilderzeugung (Markierung) kann auch manuell mit einer erhitzten Feder erfolgen. Eine weitere Einrichtung der Erzeugung von Markierungen mittels Wärme sind Laserstrahlen.

Das thermoreaktive Aufzeichnungsmaterial kann so aufgebaut sein, daß der Farbbildner in einer Bindemittelschicht gelöst oder dispergiert ist und in einer zweiten Schicht der Entwickler in dem Bindemittel gelöst oder dispergiert ist. Eine andere Möglichkeit besteht darin, daß sowohl der Farbbildner als auch der Entwickler in einer Schicht dispergiert sind. Das Bindemittel wird in spezifischen Bereichen mittels Wärme erweicht und an diesen Stellen, an denen Wärme angewendet wird, kommt der Farbbildner mit dem Elektronenakzeptor in Kontakt, und es entwickelt sich sofort die erwünschte Farbe.

Als Entwickler eignen sich die gleichen Elektronenakzeptoren wie sie in druckempfindlichen Papieren verwendet werden, vorzugsweise phenolische Verbindungen, wie sie beispielsweise in der DE-PS-1 251 348 beschrieben sind, sowie Borsäure und organische, vorzugsweise aliphatische Dicarbonsäuren.

Vorzugsweise werden zur Herstellung des thermoreaktiven Aufzeichnungsmaterials schmelzbare, filmbildende Bindemittel verwendet. Diese Bindemittel sind normalerweise wasserlöslich, während die 4,4-Diaryl-2-oxo-benzo-3,1-oxazine und der Entwickler in Wasser schwerlöslich oder unlöslich sind. Das Bindemittel sollte in der Lage sein, den Farbbildner und den Entwickler bei Raumtemperatur zu dispergieren und zu fixieren.

Bei Einwirkung von Wärme erweicht oder schmilzt das Bindemittel, so daß der Farbbildner mit dem Entwickler in Kontakt kommt und sich eine Farbe bilden kann. Wasserlösliche oder mindestens in Wasser quellbare Bindemittel sind z. B. hydrophile Polymerisate, wie Polyvinylalkohol, Polyacrylsäure, Hydroxyethylcellulose Methylcellulose, Carboxymethylcellulose, Polyacrylamid, Polyvinylpyrrolidon, Gelatine und Stärke.

Die thermoreaktiven Schichten können weitere Zusätze enthalten: zur Verbesserung des Weißgrades, zur Erleichterung des Bedruckens der Papiere, zur Verhinderung des Festklebens der erhitzten Feder und zur Farbbildung nur innerhalb eines begrenzten Temperaturbereiches.

Die beschriebenen Verfahren und Zubereitungen sind beispielsweise aus den US-Patentschriften 2 948 753, 3 096 189 und 3 193 404 und aus den deutschen Offenlegungsschriften 2 555 080 und 2 700 937 bekannt.

Aus C. A. 101 : 55014s (1984) sind aminogruppenfreie 4,4-Diphenyl-2-oxo-benzo-3,1-oxazine bekannt.

Aus der US-A-4 074 050 sind 4,4-Diphenyl-2-oxo-benzo-1,3-oxazine bekannt, die Isomere der erfindungsgemäßen chromogenen Verbindungen darstellen.

**Beispiel 1**

26,8 g Michlers Keton, 10,8 g 3-Dimethylaminophenylcarbaminsäureethylester, 76,5 g Phosphoroxychlorid und 28,2 g Diphosphorpentoxid werden 48 h bei Raumtemperatur verrührt. Anschließend wird auf 500 g Eiswasser ausgetragen, die wäßrige Lösung vom ausgefallenen Öl. abgegossen, in 100 ml Eisessig gelöst, mit 500 ml Wasser verdünnt und unter Kühlung mit NaOH als Carbinolbase gefällt. Diese Carbinolbase wird in 750 ml Dimethoxyethan gelöst, mit 15 ml konz. NaOH versetzt und 5 h bei Raumtemperatur verrührt. Die organische Phase wird abgetrennt, im Vakuum eingedampft und der Rückstand mit Methanol bis zur Kristallisation verrührt. Man erhält 35 g Produkt der Formel

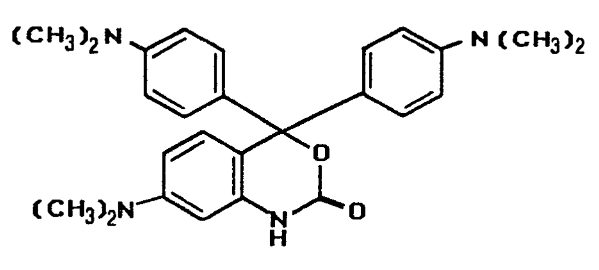

In Eisessig bildet dieses Produkt einen Farbstoff mit einer maximalen Intensität bei $\lambda_{max}$ = 596 nm.

In analoger Weise wurden folgende Beispiele hergestellt:

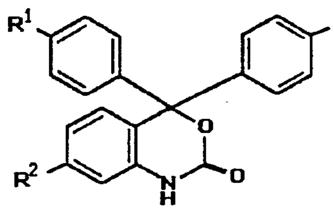

| Beispiel | $R^1$ | $R^2$ | $R^3$ | $\lambda_{max}$ |
|---|---|---|---|---|
| 2 | $-N(C_2H_5)_2$ | $-N(CH_3)_2$ | $-N(C_2H_5)_2$ | 594 nm |
| 3 | $-N(C_4H_9)_2$ | $-N(CH_3)_2$ | $-N(C_2H_9)_2$ | 597 nm |
| 4 | $-N(C_{12}H_{25})_2$ | $-N(CH_3)_2$ | $-N(CH_3)_2$ | 595 nm |

**Beispiel 5**

25,5 g 4-Dimethylamino-4'-methoxy-benzophenon, 20,8 g 3-Dimethylaminophenylcarbaminsäureethylester, 76,5 g Phosphoroxychlorid und 28,2 g Phosphorpentoxid werden 48 h bei Raumtemperatur verrührt. Man trägt auf 500 g Eiswasser aus, dekantiert von dem ausgeschiedenen Farbstofföl, löst in 100 ml Eisessig, verdünnt mit 500 ml Wasser und fällt unter Kühlung mit NaOH die Carbinolbase aus. Diese wird in 300 ml Dimethoxyethan gelöst, mit 8 ml konz. NaOH versetzt und 5 h bei Raumtemperatur verrührt. Die abgetrennte organische Phase wird im Vakuum eingedampft und mit 100 ml Methanol bis zur Kristallisation verrührt. Man erhält 30 g Produkt der Formel

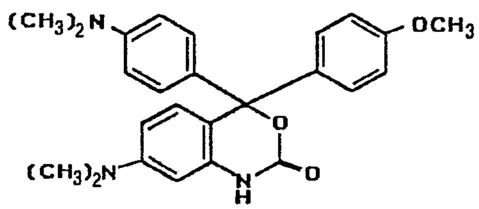

das in Essigsäure einen Farbstoff mit einer maximalen Absorption bei $\lambda_{max}$ 622 nm bildet.

In analoger Weise wurden hergestellt:

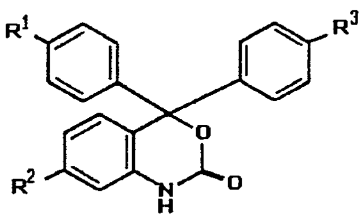

| Beispiel | $R^1$ | $R^2$ | $R^3$ | $\lambda_{max}$(nm) |
|---|---|---|---|---|
| 6 | $-N(C_2H_5)_2$ | $-N(CH_3)_2$ | $-OCH_3$ | 625 |
| 7 | $-N(CH_3)_2$ | " | $-OC_8H_{17}$ | 623 |
| 8 | $-N(C_2H_5)_2$ | " | " | 620 |
| 9 | $-N(CH_3)_2$ | " | $-OC_{16}H_{33}$ | 622 |
| 10 | 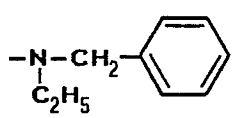 | " | $-OCH_3$ | 624 |
| 11 | $-N(C_2H_5)_2$ | " | -Cl | 642 |
| 12 | 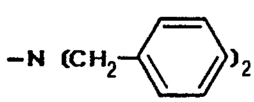 | " | $-CH_3$ | 634 |
| 13 | 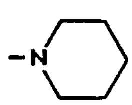 | " | $-OCH_3$ | 623 |

| Beispiel | $R^1$ | $R^2$ | $R^3$ | $\lambda_{max}$(nm) |
|---|---|---|---|---|
| 14 | 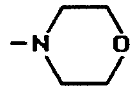 | " | -Cl | 644 |
| 15 | 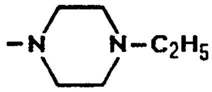 | " | -Cl | 642 |
| 16 | 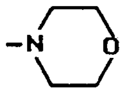 | $N(CH_3)_2$ | H | 635 |
| 17 | 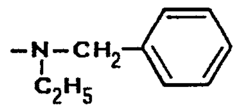 | " | Cl | 644 |
| 18 | $C_2H_5$<br>-N<br>$C_2H_4Cl$ | " | Cl | 630 |
| 19 | $CH_3$<br>-N<br>$C_2H_4CN$ | " | Cl | 634 |
| 20 | $N(CH_3)_2$ | 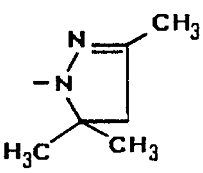 | $OCH_3$ | 646 |

**Beispiel 21**

20,8 g 3-Dimethylaminophenylcarbaminsäureethylester, 27,2 g 2,4,4'-Trimethoxybenzophenon, 76,5 g Phosphoroxychlorid und 28,2 g Phosphorpentoxid werden 48 h bei Raumtemperatur und 2 h bei 40°C verrührt. Anschließend wird auf 700 g Eiswasser ausgetragen, mit NaCl ausgesalzen, vom abgeschiedenen Farbstofföl dekantiert; das Öl wird in 300 ml Toluol gelöst und mit 300 ml gesättigter wäßriger $NaHCO_3$-Lösung verrührt, bis es in der Toluolphase gelöst ist. Die Toluolphase wird abgetrennt und eingedampft. Der verbleibende Rückstand wird in 750 ml Dimethoxyethan gelöst und mit 8 ml konz. NaOH 5 h bei Raumtemperatur verrührt. Die organische Phase wird abgetrennt, im Vakuum eingedampft und mit 100 ml Ethanol zur Kristallisation gerührt. Man erhält 40 g Produkt der Formel

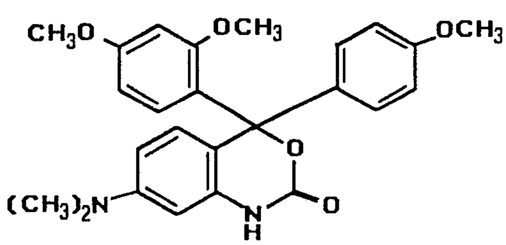

Es bildet in Essigsäure einen Farbstoff mit einer max. Absorption bei $\lambda_{max}$ = 573 nm.

**Beispiel 22**

36,7 g 4-Diethylamino-3'-methyl-benzophenon, 20,8 g Dimethylaminophenylcarbaminsäureethylester, 76,5 g Phosphoroxychlorid und 28,2 g Phosphorpentoxid werden 48 h bei Raumtemperatur verrührt. Man trägt auf 500 ml Eiswasser aus und arbeitet wie in Beispiel 5 beschrieben auf. Man erhält 35,2 g eines farblosen Produktes der Formel

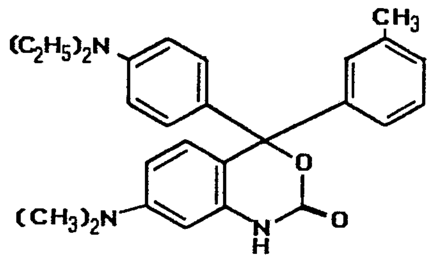

das in Eisessig einen Farbstoff mit einer max. Absorption bei $\lambda_{max}$ = 632 nm bildet.

**Beispiel 23**

28,3 g 2-Methoxy-4'-diethylamino-benzophenon, 20,8 g 3-Dimethylaminophenylcarbaminsäureethylester, 76,5 g Phosphoroxychlorid und 28,2 g Phosphorpentoxid werden 48 h bei Raumtemperatur verrührt. Man trägt auf 500 g Eiswasser aus, puffert mit Natriumacetat auf pH 6 bis 7 und fällt den Farbstoff durch Zusatz von NaCl aus. Das abgeschiedene Öl wird wie in Beispiel 5 beschrieben aufgearbeitet. Man erhält 32 g eines farblosen Produktes der Formel

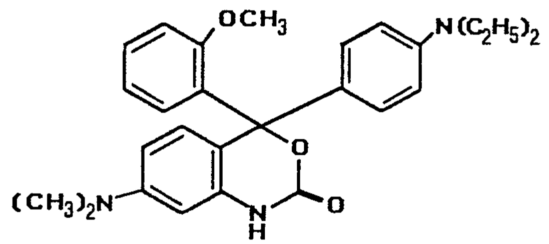

Das Produkt bildet in Eisessig einen Farbstoff mit einer maximalen Intensität bei $\lambda_{max}$ = 644 nm.

**Beispiel 24**

36,1 g 4-Methoxy-4'-(N-methyl-N-4-ethoxy-phenyl)-aminobenzophenon, 28,8 g 3-Dimethylaminophenylcarbaminsäureethylester, 76,5 g Phosphoroxychlorid und 28,1 g Phosphorpentoxid werden 48 h bei Raumtemperatur verrührt, auf 300 g Eiswasser ausgetragen und wie in Beispiel 5 beschrieben aufgearbeitet. Man erhält 43 g Produkt der Formel

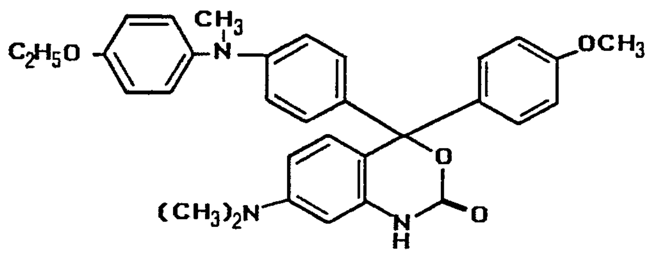

Das Produkt bildet in Eisessig einen Farbstoff mit einer maximalen Absorption bei $\lambda_{max}$ = 635 nm.

In analoger Weise wurden folgende Beispiele hergestellt:

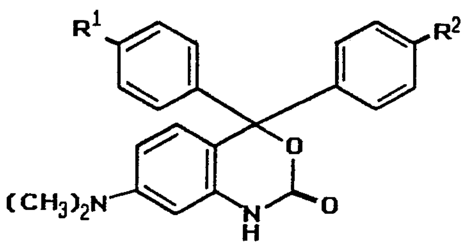

| Beispiel | $R^1$ | $R^2$ | $\lambda_{max}$(nm) |
|---|---|---|---|
| 25 | 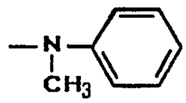 | -Cl | 629 |
| 26 | 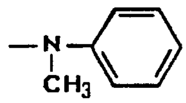 | -N | 625 |
| 27 | " | $-OCH_3$ | 616 |
| 28 | 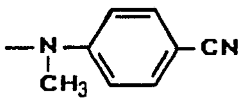 | $-OCH_3$ | 604 |

**Beispiel 29**

9,45 g der Carbinolbase des Farbstoffes der Formel

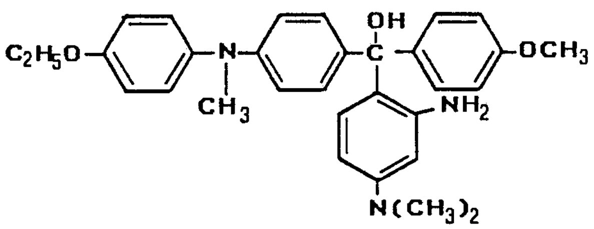

4,2 g $Na_2CO_3$ und 100 ml Ethylenglykoldimethylether werden auf 0°C gekühlt und bis zur beginnenden Färbung mit Phosgen versetzt. Man rührt 12 h nach, saugt ab, dampft im Vakuum bei Raumtemperatur ein und rührt mit 50 ml Methanol aus. Man erhält 6 g des Farbbildners aus Beispiel 19.

**Beispiel 30**

9,45 g der in Beispiel 24 als Ausgangsprodukt eingesetzten Carbinolbase werden in 50 ml Pyridin gelöst, auf 0°C gekühlt und mit Phosgen versetzt. Man rührt 12 h bei Raumtemperatur nach, trägt auf 500 ml Wasser aus und saugt ab. Ausbeute: 6,5 g Farbbildner gemäß Beispiel 19.

**Beispiel 31**

25,5 g 4-Dimethylamino-4'-methoxy-benzophenon, 20,9 g 3-Methoxy-4-methyl-phenylcarbaminsäureethylester, 76,5 g Phosphoroxychlorid und 28,2 g Phosphorpentoxid werden 48 h bei Raumtemperatur verrührt. Man trägt auf 500 ml Eiswasser aus und arbeitet wie in Beispiel 5 beschrieben auf. Man erhält 33 g des Farbbildners der Formel

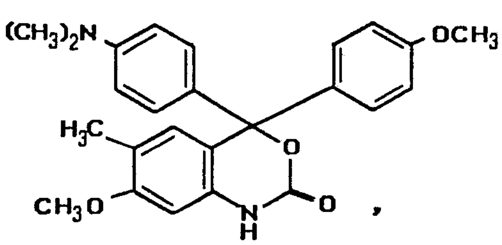,

der in Eisessig einen Farbstoff mit einer maximalen Absorption bei $\lambda_{max}$ 545 nm bildet.

In analoger Weise wurden hergestellt:

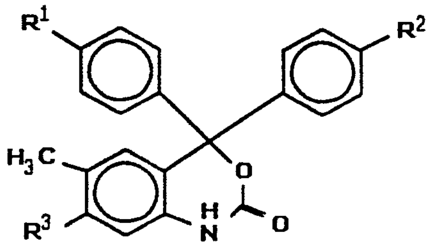

| Beispiel | $R^1$ | $R^2$ | $R^3$ | $\lambda_{max}$(nm) |
|---|---|---|---|---|
| 32 | $N(C_4H_9)_2$ | $OCH_3$ | $OCH_3$ | 610 |
| 33 | $N(C_2H_5)_2$ | $OCH_3$ | $OCH_3$ | 608 |
| 34 | $N(CH_3)_2$ | $OCH_3$ | $OC_{12}H_{25}$ | 608 |
| 35 | $N(C_3H_7)_2$ | $OCH_3$ | $OCH_3$ | 604 |
| 36 | $N(C_3H_7)_2$ | $OC_2H_5$ | $OCH_3$ | 606 |
| 37 | $CH_3$ \| N \| $C_2H_4CN$ | $OCH_3$ | $OCH_3$ | 598 |
| 38 | $N(C_2H_5)_2$ | $OC_3H_7$ | $OCH_3$ | 604 |
| 39 | $N(CH_3)_2$ | $N(CH_3)_2$ | H | 628 |

**Beispiel 40**

**Herstellung eines druckempfindlichen Kopierpapiers**

Eine Lösung von 3 g des Farbbildners aus Beispiel 18 in 80 g Diisopropylnaphthalin und 17 g Kerosin wird auf an sich bekannte Weise mit Gelatine und Gummiarabicum durch Koazervation mikroverkapselt, mit Stärkelösung vermischt und auf ein Blatt Papier gestrichen. Ein zweites Blatt Papier wird auf der Frontseite mit säureaktiviertem Bentonit als Farbentwickler beschichtet. Das erste Blatt und das mit Farbentwickler beschichtete Blatt werden mit den Beschichtungen benachbart aufeinandergelegt. Durch Schreiben mit der Hand oder mit der Schreibmaschine auf dem ersten Blatt wird Druck ausgeübt, und es entwickelt sich auf dem mit dem Entwickler beschichteten Blatt eine intensive blau-grüne Kopie, die ausgezeichnet lichtecht ist.

**Beispiel 41**

1 g der Farbbilderns des Beispiels 11 wird in 17 g Toluol gelöst. Zu dieser Lösung gibt man unter Rühren 12 g Polyvinylacetat, 8 g Calciumcarbonat und 2 g Titandioxid. Die erhaltene Suspension wird im Gewichtsverhältnis 1/1 mit Toluol verdünnt und mit einem 10 µm Rakel auf ein Blatt Papier gestrichen. Auf dieses Blatt Papier wird ein zweites Blatt Papier gelegt, dessen Unterseite bei einem Auftragsgewicht von 3 g/m mit einer Mischung - bestehend aus 1 Teil eines Amidwachses, 1 Teil eines Stearinwachses und 1 Teil Zinkchlorid - beschichtet ist. Durch Schreiben mit der Hand oder mit der Schreibmaschine auf dem oberen Blatt wird Druck ausgeübt, und es entwickelt sich auf dem mit dem Farbbildner beschichteten Blatt eine intensive und lichtechte grüne Farbe.

**Beispiel 42**

**Herstellung eines wärmeempfindlichen Aufzeichnungsmaterials**

In einer Kugelmühle werden 32 g 4,4'-Isopropyliden-diphenol (Bisphenol A), 3,8 g Distearylamid des Ethylendiamins, 89 g Kaolin, 20 g eines zu 88 % hydrolysierten Polyvinylalkohols und 55 ml Wasser gemahlen, bis die Teilchengröße ca. 5 µm beträgt. In einer 2. Kugelmühle werden 6 g der Verbindung des Beispiels 19,3 g eines zu 88 % hydrolysierten Polyvinylalkohols und 60 ml Wasser zu einer Teilchengröße von ca. 3 µm gemahlen. Die beiden Dispersionen werden zusammengegeben und mit einem Trockenauftragsgewicht von 5,5 $g/m^2$ auf ein Papier gestrichen. Durch Berühren des Papiers mit einem erhitzten Kugelschreiber wird eine intensive grünschwarze Farbe erhalten, die eine gute Licht- und Sublimierechtheit hat.

**Beispiel 43**

In einer Kugelmühle werden 2,7 g der Verbindung des Beispiels 23, 24 g N-Phenyl-N'-(1-hydroxy-2,2,2-trichlor-ethyl)-harnstoff, 16 g Stearinsäureamid, 59 g eines zu 88 % hydrolysierten Polyvinylalkohols und 58 ml Wasser gemahlen, bis die Teilchengröße 2 - 5 µm beträgt. Diese Suspension wird bei einem Trockenauftragsgewicht von 5,5 g/m$^2$ auf ein Blatt Papier gestrichen. Durch Berührung des Papiers mit einem erhitzten Kugelschreiber wird eine intensive und lichtechte schwarze Farbe erhalten.

## Patentansprüche

1. Chromogene Verbindungen der allgemeinen Formel

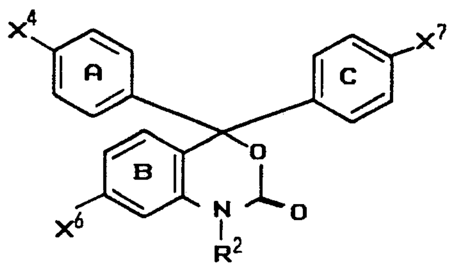

worin

$X^4$, $X^6$ und $X^7$ unabhängig voneinander Wasserstoff, Halogen, $C_1$- bis $C_{18}$-Alkyl, gegebenenfalls durch Chlor und/oder $C_1$- bis $C_{12}$-Alkyl substituiertes Phenyl, $C_1$- bis $C_{12}$-Alkylcarbonylamino, gegebenenfalls durch Chlor und/oder $C_1$- bis $C_{12}$-Alkyl substituiertes Benzoylamino, $NY^4Y^5$, $OY^6$ oder $SY^6$, 2- oder 4-Pyridyl , 2-, 4- oder 5-Pyrimidyl, Pyrazolinyl, 3-Indolyl oder 4-Piperidyl, die durch $C_1$- bis $C_4$-Alkyl oder Phenyl substitueirt sein können, wobei Phenyl seinerseits durch Amino, $C_1$- bis $C_{12}$-Mono- oder -Dialkylamino, Halogen, $C_1$- bis $C_{12}$ -Alkoxy oder $C_1$-$C_{12}$-Alkyl substituiert sein kann,

wobei wenigstens einer der Reste

$X^4$, $X^6$ oder $X^7$ für $NY^4Y^5$ steht,
$R^2$ Wasserstoff, $C_1$- bis $C_4$-Alkyl, Cyclohexyl oder gegebenenfalls durch Chlor und/oder $C_1$- bis $C_4$-Alkyl substituierte Benzyl- oder Phenylreste,
$Y^4$, $Y^5$ und $Y^6$ unabhängig voneinander Wasserstoff, $C_1$-$C_{18}$-Alkyl, das durch Chlor, Cyano, $C_1$- bis $C_{12}$-Alkoxycarbonyl oder $C_1$- bis $C_{12}$-Alkoxy substituiert sein kann, Cyclohexyl, Phenyl oder Benzyl, die durch Chlor, Hydroxy, $C_1$- bis $C_{12}$-Alkyl oder $C_1$- bis $C_{12}$-Alkoxy,

substituiert sein können, oder Glieder, die zusammen mit N oder O, an das sie gebunden sind, und mit einem der Ringe A, B oder C zur Vervollständigung eines Ringsystems der folgenden Formeln

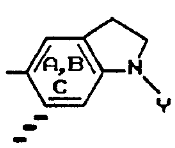 , 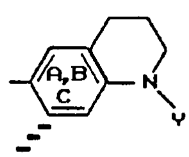 , 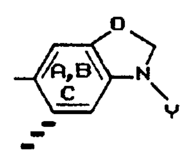 , 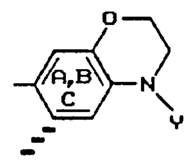 ,

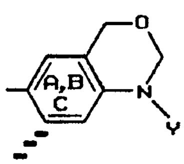 , 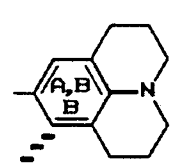 , 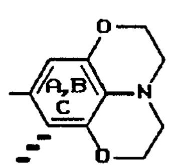 , 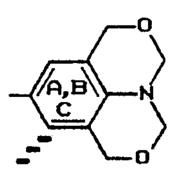 ,

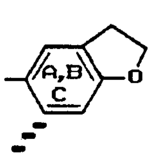 , 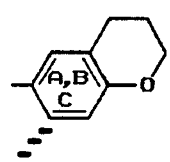 , 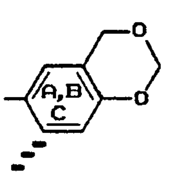 , 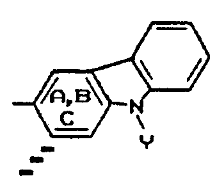

erforderlich sind, bedeuten, worin

die gestrichelte Linie die weitere Anellierung im Fall von Ring B bedeutet,

Y für Wasserstoff, gegebenenfalls durch Chlor, Cyano, $C_1$- bis $C_4$-Alkoxycarbonyl oder $C_1$- bis $C_4$-Alkoxy substituiertes $C_1$ bis $C_8$-Alkyl, Cyclohexyl oder Phenyl oder Benzyl, die durch Chlor, $C_1$- bis $C_4$-Alkyl oder $C_1$- bis $C_4$-Alkoxy substituiert sein können, steht,

der gesättigte Ringteil bis zu 4 Reste aus der Gruppe Chlor, $C_1$- bis $C_4$-Alkyl, $C_1$- bis $C_4$-Alkoxy und Phenyl tragen kann,
die Ringe A, B, C durch Chlor, $C_1$- bis $C_4$-Alkyl, $C_1$- bis $C_4$-Alkoxy und/oder $C_1$- bis $C_{12}$-Alkanoylamino substituiert sein können, oder
$NY^4Y^5$ einen gegebenenfalls durch Chlor, $C_1$- bis $C_{18}$-Alkyl, Amino-$C_1$-$C_4$-Alkyl oder Phenyl substituierten Pyrrolo-, Pyrrolidino-, Piperidino-, Pipecolino-, Morpholino-, Pyrazolo- oder Pyrazolinorest bedeuten.

2. Chromogene Verbindungen gemäß Anspruch 1 der allgemeinen Formel

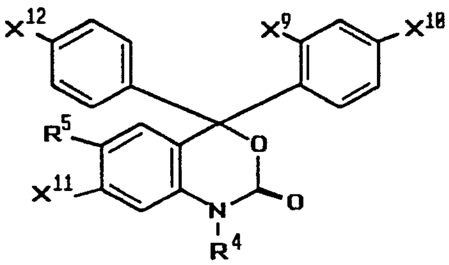

worin wenigstens einer der Reste

$X^{10}$, $X^{11}$ oder $X^{12}$ $NY^7Y^{7'}$ und die übrigen unabhängig voneinander $NY^7Y^{7'}$, Wasserstoff, Halogen, $C_1$ - bis $C_{18}$-Alkyl, $C_1$- bis $C_{12}$-Alkoxy, Benzyloxy, Methyl- oder Ethylmercapto, Phenyl oder Pyrazolino, das durch $C_1$- bis $C_4$-Alkyl oder Phenyl substituiert sein kann,

$X^9$ Wasserstoff, $C_1$- bis $C_4$-Alkyl, $C_1$- bis $C_4$-Alkoxy, Benzyloxy, Methyl- oder Ethylmercapto,

$Y^7$ und $Y^{7'}$ unabhängig voneinander Wasserstoff, $C_1$- bis $C_{18}$-Alkyl, das durch Cyan, Chlor, $C_1$- bis $C_3$-Alkoxy oder $C_1$- bis $C_3$-Alkoxycarbonyl substituiert sein kann oder dessen Substituenten zu einem Piperidin-, Morpholin-, Piperazinring geschlossen sein können, Benzyl oder Phenyl, die durch Chlor, Hydroxy, $C_1$- bis $C_4$-Alkyl, $C_1$- bis $C_4$-Alkoxy, Cyan oder $C_1$- bis $C_4$-Alkoxy-carbonyl substituiert sein können, bedeuten, und
$R^4$ und $R^5$ unabhängig voneinander für Wasserstoff, $C_1$-bis $C_4$-Alkyl, Cyclohexyl, Benzyl oder Phenyl stehen.

3. Chromogene Verbindungen gemäß Anspruch 1 der allgemeinen Formel

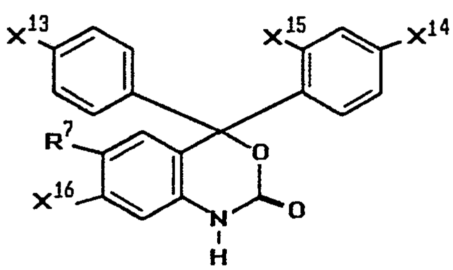

worin

$R^7$ Wasserstoff, $C_1$- bis $C_4$-Alkyl oder Benzyl bedeutet,

wenigstens einer der Reste

$X^{13}$, $X^{14}$ oder $X^{16}$ für $NY^9Y^{9'}$ steht und die übrigen unabhängig voneinander Wasserstoff, $NY^9Y^{9'}$, Halogen, $C_1$-$C_{18}$-Alkyl, $C_1$-$C_{12}$-Alkoxy, Benzyloxy, Methylmercapto oder Ethylmercapto, Phenyl oder Pyrazolino, das durch $C_1$- bis $C_4$-Alkylgruppen oder Phenylgruppen weiter substituiert sein kann,

$X^{15}$ Wasserstoff, $C_1$- bis $C_3$-Alkoxy, Methylmercapto oder Ethylmercapto, $Y^9$ und $Y^{9'}$ unabhängig voneinander Wasserstoff, $C_1$-bis $C_{18}$-Alkyl, das durch Cyano, Chlor, $C_1$- bis $C_3$-Alkoxy oder $C_1$- bis $C_3$-Alkoxycarbonyl substituiert sein kann, oder Benzyl, das durch Chlor, $C_1$- bis $C_4$-Alkyl oder $C_1$- bis $C_4$-Alkoxy, substituiert sein kann, oder

$Y^{9'}$ einen Rest der Formel, 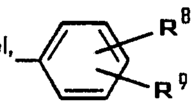 bedeuten,

worin

$R^8$ und $R^9$ unabhängig voneinander Chlor, $C_1$- bis $C_4$-Alkyl, $C_1$- bis $C_4$-Alkoxy, Phenyloxy, Benzyloxy, Cyano, Pyrrolidino, Piperidino, Morpholino, Piperazino, Pyrazolino oder Imidazolino bedeuten.

4. Farbstoffe der allgemeinen Formel

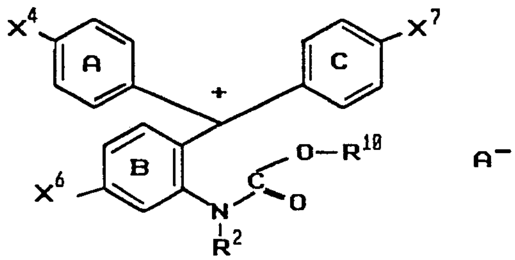

worin

$X^4$, $X^6$, $X^7$, $R^2$, A, B und C die in Anspruch 1 angegebene Bedeutung haben,

$A^-$ ein Anion bedeutet und

$R^{10}$ für $C_1$-$C_4$-Alkyl oder Phenyl steht.

5. Verfahren zur Herstellung der Farbbildner des Anspruchs 1, dadurch gekennzeichnet, daß man die Farbstoffe des Anspruches 4 in wäßrigem und/oder nicht-wäßrigem Medium unter Einwirkung einer Base cyclisiert.

6. Verfahren zur Herstellung der Farbbildner des Anspruchs 1, dadurch gekennzeichnet, daß man Farbstoffe der allgemeinen Formel

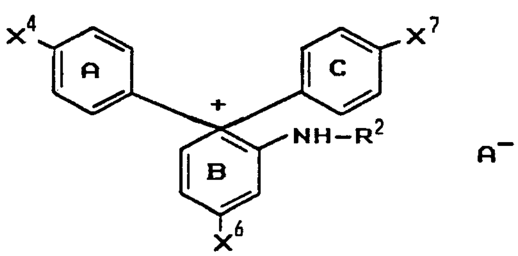

worin

$X^4$, $X^6$, $X^7$ und $R^2$ die in Anspruch 1 aufgeführte Bedeutung haben,

$A^-$ für ein Anion steht und die Ringe

A, B und C mit in der Farbstoffchemie üblichen nichtionischen Substituenten weiter substituiert sein können,

mit Phosgen oder seinen Derivaten in Gegenwart einer Base umsetzt.

7. Verwendung von 4,4-Diaryl-2-oxo-benzo-3,1-oxazine gemäß Anspruch 1 als Farbbildner in einem druck- oder wärmeempfindlichen Aufzeichnungsmaterial.

8. Druck- oder wärmeempfindliches Aufzeichnungsmaterial, dadurch gekennzeichnet, daß es in seinem Reaktantensystem mindestens eine Verbindung des Anspruchs 1 als Farbbildner enthält.

9. Leukoverbindungen der Formel

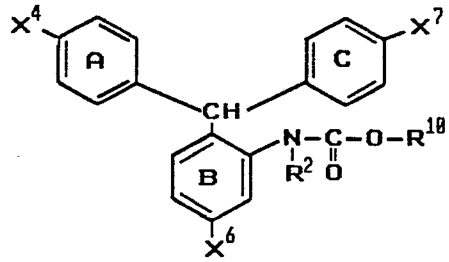

worin

$X^4$, $X^6$, $X^7$, $R^2$, A, B und C die in Anspruch 1 angegebene Bedeutung haben und

$R^{10}$ die in Anspruch 4 aufgeführte Bedeutung besitzt.

## Claims

1. Chromogenic compounds of the general formula

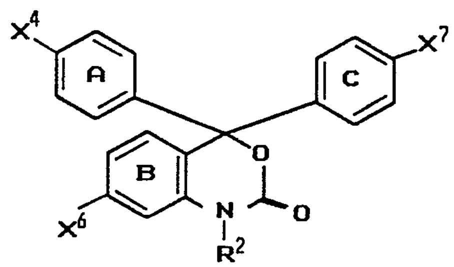

wherein

$X^4$, $X^6$ and $X^7$, independently of each other, denote hydrogen, halogen, $C_1$- to $C_{18}$-alkyl, optionally chlorine- and/or $C_1$- to $C_{12}$-alkyl-substituted phenyl, $C_1$- to $C_{12}$-alkylcarbonylamino, optionally chlorine and/or $C_1$- to $C_{12}$-alkyl-substituted benzoylamino, $NY^4Y^5$, $OY^6$ or $SY^6$, 2- or 4-pyridyl, 2-, 4- or 5-pyrimidyl, pyrazolinyl, 3-indolyl or 4-piperidyl, which can be substituted by $C_1$- to $C_4$-alkyl or phenyl, it being possible for phenyl in turn to be substituted by amino, $C_1$- to $C_{12}$ -mono- or -dialkylamino, halogen, $C_1$- to $C_{12}$-alkoxy or $C_1$-$C_{12}$-alkyl,

wherein at least one of the radicals

$X^4$, $X^6$ or $X^7$ represents $NY^4Y^5$,

$R^2$ represents hydrogen, $C_1$- to $C_4$-alkyl, cyclohexyl or optionally chlorine- and/or $C_1$ - to $C_4$-alkylsubstituted benzyl or phenyl radicals,

$Y^4$, $Y^5$ and $Y^6$, independently of each other, denote hydrogen, $C_1$-$C_{18}$-alkyl, which can be substituted by chlorine, cyano, $C_1$- to $C_{12}$-alkoxycarbonyl or $C_1$ - to $C_{12}$-alkoxy, cyclohexyl, phenyl or benzyl, which can be substituted by chlorine, hydroxyl, $C_1$- to $C_{12}$-alkyl or $C_1$- to $C_{12}$-alkoxy, or members which, together with N or O, to which they are bonded, and with one of the rings A, B or C, are necessary for completing a ring system of the following formulae

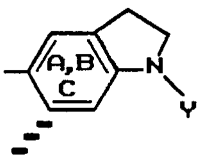 , 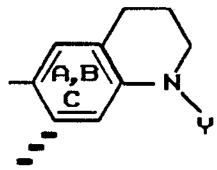 , 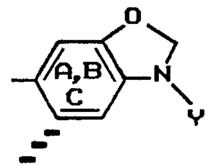 , 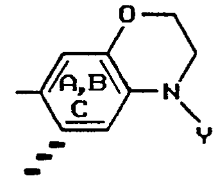

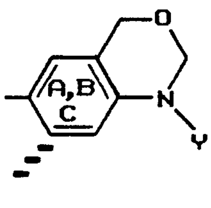 , 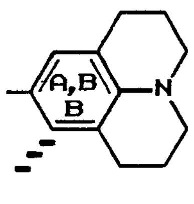 , 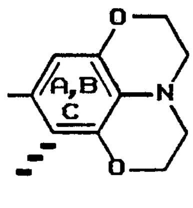 , 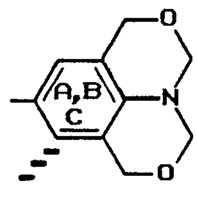 ,

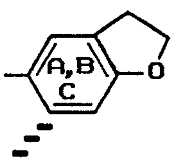 , 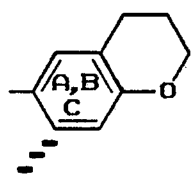 , 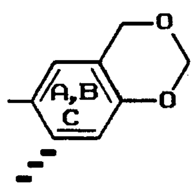 , 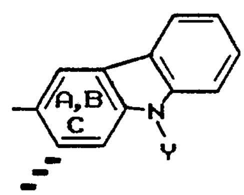

wherein

the broken line denotes the further fusion in the case of ring B,

Y represents hydrogen, optionally chlorine-, cyano-, $C_1$ - to $C_4$-alkoxycarbonyl- or $C_1$- to $C_4$-alkoxy-substituted $C_1$- to $C_8$-alkyl, cyclohexyl or phenyl or benzyl, which can be substituted by chlorine, $C_1$ - to $C_4$-alkyl or $C_1$ - to $C_4$-alkoxy,
the saturated ring moiety can carry up to 4 radicals from the group comprising chlorine, $C_1$- to $C_4$-alkyl, $C_1$ - to $C_4$-alkoxy and phenyl,
the rings A, B and C can be substituted by chlorine, $C_1$- to $C_4$-alkyl, $C_1$- to $C_4$-alkoxy and/or $C_1$- to $C_{12}$-alkanoylamino, or $NY^4Y^5$ denotes an optionally chlorine-, $C_1$- to $C_{18}$-alkyl-, amino-$C_1$-$C_4$-alkyl- or phenyl-substituted pyrrolo, pyrrolidino, piperidino, pipecolino, morpholino, pyrazolo or pyrazolino radical.

2. Chromogenic compounds according to Claim 1 of the general formula

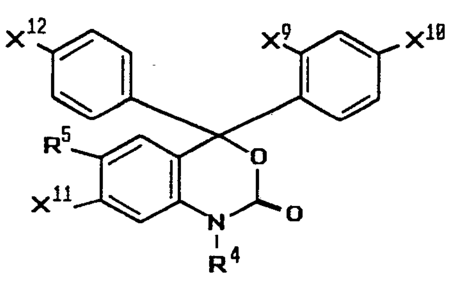

wherein

at least one of the radicals

$X^{10}$, $X^{11}$ or $X^{12}$ denotes $NY^7Y^{7'}$ and the others, independently of each other, denote $NY^7Y^{7'}$ hydrogen, halogen, $C_1$- to $C_{18}$-alkyl, $C_1$- to $C_{12}$-alkoxy, benzyloxy, methyl- or ethylmercapto, phenyl or pyrazolino, which can be substituted by $C_1$- to $C_4$-alkyl or phenyl,

$X^9$ denotes hydrogen, $C_1$- to $C_4$-alkyl, $C_1$- to $C_4$-alkoxy, benzyloxy, methyl- or ethylmercapto,

$Y^7$ and $Y^{7'}$, independently of each other, denote hydrogen, $C_1$- to $C_{18}$-alkyl, which can be substituted by cyano, chlorine, $C_1$- to $C_3$-alkoxy or $C_1$ - to $C_3$-alkoxycarbonyl or its substituents can be cyclised to form a piperidine, morpholine or

piperazine ring, benzyl or phenyl, which can be substituted by chlorine, hydroxyl, $C_1$- to $C_4$-alkyl, $C_1$- to $C_4$-alkoxy, cyano or $C_1$- to $C_4$-alkoxycarbonyl, and

$R^4$ and $R^5$, independently of each other, represent hydrogen, $C_1$- to $C_4$-alkyl, cyclohexyl, benzyl or phenyl.

3. Chromogenic compounds according to Claim 1 of the general formula

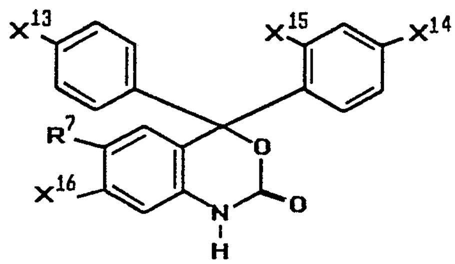

wherein

$R^7$ denotes hydrogen, $C_1$- to $C_4$-alkyl or benzyl, at least one of the radicals

$X^{13}$, $X^{14}$ or $X^{16}$ represents $NY^9Y^{9'}$ and the others, independently of each other, denote hydrogen, $NY^9Y^{9'}$, halogen, $C_1$-$C_{18}$-alkyl, $C_1$-$C_{12}$-alkoxy, benzyloxy, methylmercapto or ethylmercapto, phenyl or pyrazolino, which can be further substituted by $C_1$- to $C_4$-alkyl groups or phenyl groups,

$X^{15}$ denotes hydrogen, $C_1$- to $C_3$-alkoxy, methylmercapto or ethylmercapto,

$Y^9$ and $Y^{9'}$, independently of each other, denote hydrogen, $C_1$- to $C_{18}$-alkyl which can be substituted by cyano, chlorine, $C_1$- to $C_3$-alkoxy or $C_1$- to $C_3$-alkoxycarbonyl, or benzyl which can be substituted by chlorine, $C_1$- to $C_4$-alkyl, or $C_1$- to $C_4$-alkoxy, or $Y^{9'}$ denotes a radical of the formula

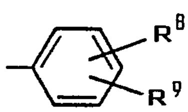

wherein

$R^8$ and $R^9$, independently of each other, denote chlorine, $C_1$- to $C_4$-alkyl, $C_1$- to $C_4$-alkoxy, phenyloxy, benzyloxy, cyano, pyrrolidino, piperidino, morpholino, piperazino, pyrazolino or imidazolino.

4. Dyestuffs of the general formula

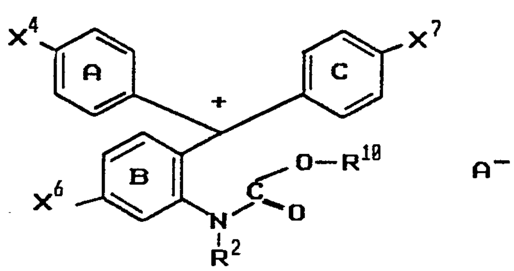

wherein

$X^4$, $X^6$, $X^7$, $R^2$, A, B and C have the meaning given in Claim 1,

$A^-$ denotes an anion and

$R^{10}$ represents $C_1$-$C_4$-alkyl or phenyl.

5. Process for preparing the colour formers of Claim 1, characterised in that the dyestuffs of Claim 4 are cyclised in an aqueous and/or non-aqueous medium under the action of a base.

6. Process for preparing the colour formers of Claim 1, characterised in that dyestuffs of the general formula

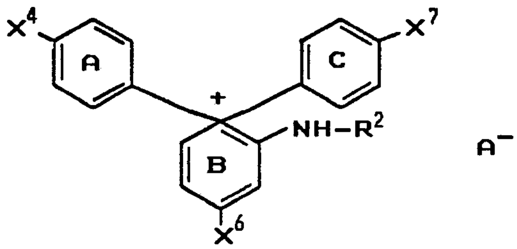

wherein

$X^4$, $X^6$, $X^7$ and $R^2$ have the meaning given in Claim 1,

$A^-$ represents an anion and the rings
A, B and C can be further substituted with non-ionic substituents customary in dyestuff chemistry,
are reacted with phosgene or its derivatives in the presence of a base.

7. Use of 4,4-diary-2-oxo-benzo-3,1-oxazines according to Claim 1 as colour formers in a pressure- or heat-sensitive recording material.

8. Pressure- or heat-sensitive recording material, characterised in that it contains in its reactant system at least one compound of Claim 1 as a colour former.

9. Leuco compounds of the formula

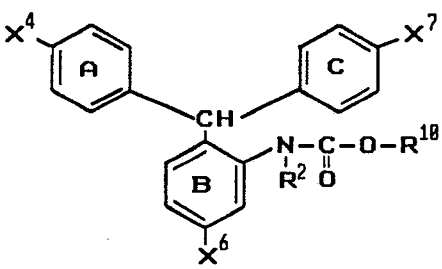

wherein

$X^4$, $X^6$, $X^7$, $R^2$, A, B and C have the meaning given in Claim 1 and

$R^{10}$ has the meaning mentioned in Claim 4.

**Revendications**

1. Composés chromogènes de formule générale

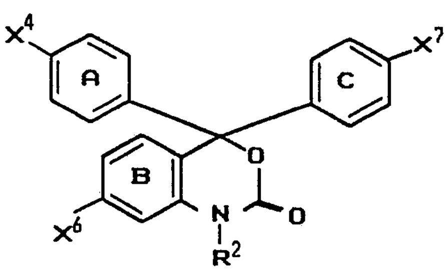

dans laquelle

$X^4$, $X^6$ et $X^7$ représentent chacun, indépendamment les uns des autres, l'hydrogène, un halogène un groupe alkyle en $C_1$-$C_{18}$, un groupe phényle éventuellement substitué par le chlore et/ou des groupes alkyle en $C_1$-$C_{12}$, un groupe

(alkyle en $C_1$-$C_{12}$)-carbonyl-amino, un groupe benzoylamino éventuellement substitué par le chlore et/ou des groupes alkyle en $C_1$-$C_{12}$, un groupe $NY^4Y^5$, $OY^6$ ou $SY^6$, un groupe 2- ou 4-pyridyle, 2-, 4- ou 5-pyrimidyle, pyrazolinyle, 3-indolyle, ou 4-pipéridyle qui peuvent être substitués par des groupes alkyle en $C_1$-$C_4$ ou phényle, le groupe phényle pouvant lui-même être substitué par des groupes amino, mono- ou di-(alkyle en $C_1$-$C_{12}$)-amino, des halogènes, des groupes alcoxy en $C_1$-$C_{12}$ ou alkyle en $C_1$-$C_{12}$, l'un au moins des symboles

$X^4$, $X^6$ ou $X^7$ représentant $NY^4Y^5$,

$R^2$ représente l'hydrogène, un groupe alkyle en $C_1$-$C_4$, cyclohexyle ou un groupe benzyle ou phényle éventuellement substitué par le chlore et/ou des groupes alkyle en $C_1$-$C_4$,

$Y^4$, $Y^5$ et $Y^6$ représentent chacun, indépendamment les uns des autres, l'hydrogène, un groupe alkyle en $C_1$-$C_{18}$ qui peut être substitué par le chlore, des groupes cyano, (alcoxy en $C_1$-$C_{12}$)-carbonyle ou alcoxy en $C_1$-$C_{12}$, un groupe cyclohexyle, phényle ou benzyle qui peuvent être substitués par le chlore, des groupes hydroxy, alkyle en $C_1$-$C_4$ ou alcoxy en $C_1$-$C_{12}$, ou des chaînons nécessaires pour compléter, avec l'atome de N ou de O auquel ils sont reliés, et avec l'un des cycles A, B ou C, un système cyclique répondant à l'une des formules suivantes:

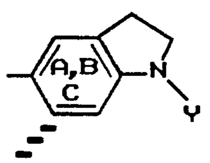 , 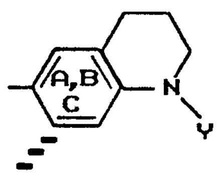 , 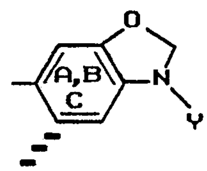 , 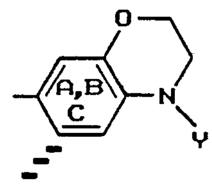

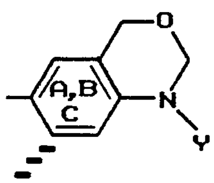 , 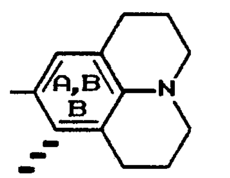 , 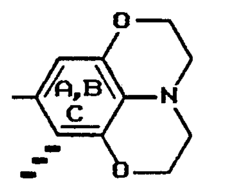 , 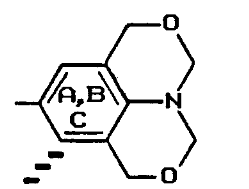 ,

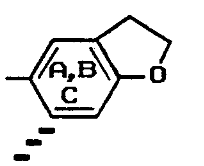 , 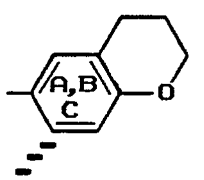 , 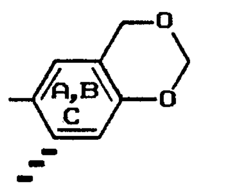 , 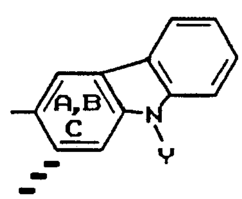

dans lesquelles

le trait en pointillé indique une autre condensation dans le cas du cycle B,

Y représente l'hydrogène, un groupe alkyle en $C_1$-$C_8$ éventuellement substitué par le chlore, des groupes cyano, (alcoxy en $C_1$-$C_4$)-carbonyle ou alcoxy en $C_1$-$C_4$, un groupe cyclohexyle ou phényle ou benzyle qui peuvent être substitués par le chlore, des groupes alkyle en $C_1$-$C_4$ ou alcoxy en $C_1$-$C_4$,
la partie cyclique saturée pouvant porter jusqu'à 4 substituants choisis dans le groupe formé par le chlore, les groupes alkyle en $C_1$-$C_4$, alcoxy en $C_1$-$C_4$ et phényle,
les cycles A, B, C peuvent être substitués par le chlore, des groupes alkyle en $C_1$-$C_4$, alcoxy en $C_1$-$C_4$ et/ou alcanoylamino en $C_1$-$C_{12}$, ou bien $NY^4Y^5$ représente un groupe pyrrolo, pyrrolidino, pipéridino, pipécolino, morpholino, pyrazolo ou pyrazolino éventuellement substitué par le chlore, des groupes alkyle en $C_1$-$C_{18}$, aminoalkyle en $C_1$-$C_4$ ou phényle.

2. Composés chromogènes selon la revendication 1, répondant à la formule générale

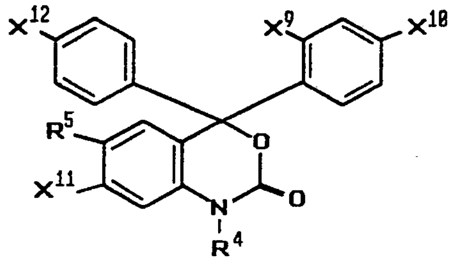

dans laquelle l'un au moins des symboles

$X^{10}$, $X^{11}$ ou $X^{12}$ représente $NY^7Y^{7'}$ et les autres, indépendamment l'un de l'autre, $NY^7Y^{7'}$, l'hydrogène, un halogène, un groupe alkyle en $C_1$-$C_{18}$, alcoxy en $C_1$-$C_{12}$, benzyloxy, méthyl- ou éthyl-mercapto, phényle ou pyrazolino qui peut être substitué par des groupes alkyle en $C_1$-$C_4$ ou phényle,

$X^9$ représente l'hydrogène, un groupe alkyle en $C_1$-$C_4$, alcoxy en $C_1$-$C_4$, benzyloxy, méthyl- ou éthyl-mercapto,

$Y^7$ et $Y^{7'}$ représentent chacun, indépendamment l'un de l'autre, l'hydrogène, un groupe alkyle en $C_1$-$C_{18}$ qui peut être substitué par des groupes cyano, le chlore, des groupes alcoxy en $C_1$-$C_3$ ou (alcoxy en $C_1$-$C_3$)-carbonyle et dont les substituants peuvent être reliés entre eux avec formation d'un cycle pipéridine, morpholine, pipérazine, un groupe benzyle ou phényle, qui peuvent être substitués par le chlore, des groupes hydroxy, alkyle en $C_1$-$C_4$, alcoxy en $C_1$-$C_4$, cyano ou (alcoxy en $C_1$-$C_4$)-carbonyle et

$R^4$ et $R^5$ représentent chacun, indépendamment l'un de l'autre, l'hydrogène, un groupe alkyle en $C_1$-$C_4$, cyclohexyle, benzyle ou phényle.

3. Composés chromogènes selon la revendication 1, répondant à la formule générale

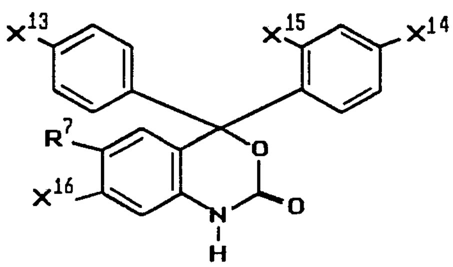

dans laquelle

$R^7$ représente l'hydrogène, un groupe alkyle en $C_1$-$C_4$ ou benzyle, l'un au moins des symboles

$X^{13}$, $X^{14}$ ou $X^{16}$ représente $NY^9Y^{9'}$ et les autres, indépendamment l'un de l'autre, l'hydrogène, un groupe $NY^9Y^{9'}$, un halogène, un groupe alkyle en $C_1$-$C_4$, alcoxy en $C_1$-$C_{12}$, benzyloxy, méthylmercapto ou éthyl-mercapto, phényle ou pyrrazolino qui peut lui-même être substitué par des groupes alkyle en $C_1$-$C_4$ ou phényle, $X^{15}$ représente l'hydrogène, un groupe alcoxy en $C_1$-$C_3$, méthylmercapto ou éthyl-mercapto,

$Y^9$ et $Y^{9'}$ représentent chacun, indépendamment l'un de l'autre, l'hydrogène, un groupe alkyle en $C_1$-$C_{18}$ qui peut être substitué par des groupes cyano, le chlore, des groupes alcoxy en $C_1$ - $C_3$ ou (alcoxy en $C_1$-$C_3$)-carbonyle, ou un groupe benzyle qui peut être substitué par le chlore, des groupes alkyle en $C_1$-$C_4$ ou alcoxy en $C_1$-$C_4$, ou bien

$Y^{9'}$ représente un groupe de formule 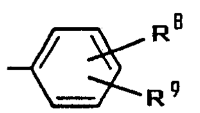

dans lequel

$R^8$ et $R^9$ représentent chacun, indépendamment l'un de l'autre, le chlore, un groupe alkyle en $C_1$-$C_4$, alcoxy en $C_1$-$C_4$, phényloxy, benzyloxy, cyano, pyrrolidino, pipéridino, morpholino, pipérazino, pyrazolino ou imidazolino.

4. Colorants de formule générale

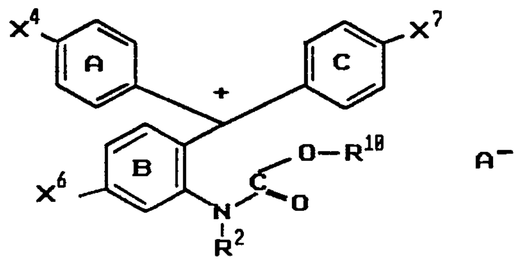

dans laquelle

$X^4$, $X^6$, $X^7$, $R^2$, A, B et C ont les significations indiquées dans la revendication 1,

$A^-$ représente un anion et

$R^{10}$ représente un groupe alkyle en $C_1$-$C_4$ ou phényle.

5. Procédé de préparation des chromogènes de la revendication 1, caractérisé en ce que l'on cyclise les colorants de la revendication 4 en milieu aqueux et/ou non aqueux sous l'action d'une base.

6. Procédé de préparation des chromogènes de la revendication 1, caractérisé en ce que l'on fait réagir des colorants de formule générale

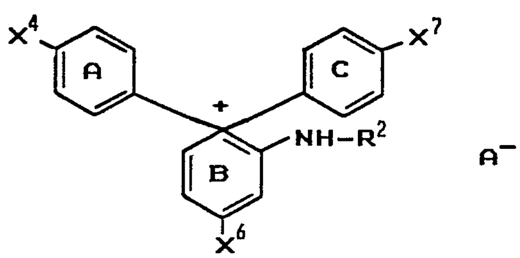

dans laquelle

$X^4$, $X^6$, $X^7$ et $R^2$ ont les significations indiquées dans la revendication 1,

$A^-$ représente un anion et les cycles
A, B et C peuvent porter d'autres substituants non ioniques usuels dans la chimie des colorants,
avec le phosgène ou ses dérivés en présence d'une base.

7. Utilisation des 4,4-diaryl-2-oxo-benzo-3,1-oxazines selon la revendication 1 en tant que chromogènes dans un matériau d'enregistrement sensible à la pression ou à la chaleur.

8. Matériau d'enregistrement sensible à la pression ou à la chaleur, caractérisé en ce qu'ils contient dans son système de réactifs au moins un composé de la revendication 1 en tant que chromogène.

9. Leucodérivés de formule

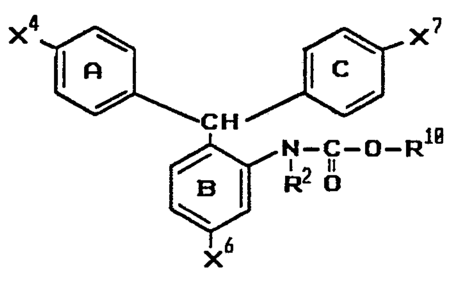

dans laquelle

$X^4$, $X^6$, $X^7$, $R^2$, A, B et C ont les significations indiquées dans la revendication 1 et

$R^{10}$ a les significations indiquées dans la revendication 4.